(19) Europäisches Patentamt
European Patent Office
Office européen des brevets

(11) **EP 1 758 612 B1**

(12) **EUROPEAN PATENT SPECIFICATION**

(45) Date of publication and mention
of the grant of the patent:
**11.06.2008 Bulletin 2008/24**

(51) Int Cl.:
***A61K 47/02*** (2006.01)

(21) Application number: **05779986.8**

(22) Date of filing: **20.05.2005**

(86) International application number:
**PCT/US2005/017726**

(87) International publication number:
**WO 2005/112887 (01.12.2005 Gazette 2005/48)**

(54) **COMPOSITION COMPRISING INTERCALATED FUNCTIONAL-ACTIVE ORGANIC COMPOUNDS**

ZUSAMMENSETZUNG MIT INTERKALIERTEN FUNKTIONELL AKTIVEN ORGANISCHEN VERBINDUNGEN

COMPOSITION COMPRENANT UN COMPOSE ORGANIQUE ACTIF FONCTIONNEL INTERCALE

(84) Designated Contracting States:
**DE GB**

(30) Priority: **20.05.2004 US 850492**

(43) Date of publication of application:
**07.03.2007 Bulletin 2007/10**

(73) Proprietor: **Eastman Kodak Company
Rochester, New York 14650-2201 (US)**

(72) Inventor: **BRINGLEY, Joseph Francis
Rochester, New York 14612 (US)**

(74) Representative: **Haile, Helen Cynthia
Kodak Limited, Patent Department
(W93-2), Headstone Drive, Harrow
Middlesex, HA1 4TY (GB)**

(56) References cited:
  EP-A- 0 987 328          US-A1- 2003 129 243
  US-A1- 2004 052 849

• KHAN A I ET AL: "Intercalation and controlled release of pharmaceutically active compounds from a layered double hydroxide." CHEMICAL COMMUNICATIONS (CAMBRIDGE, ENGLAND) 21 NOV 2001, no. 22, 21 November 2001 (2001-11-21), pages 2342-2343, XP002367498 ISSN: 1359-7345
• CHOY ET AL: "Intercalative Nanohybrids of Nucleoside Monophosphates and DNA in Layered Metal Hydroxide" JOURNAL OF THE AMERICAN CHEMICAL SOCIETY, AMERICAN CHEMICAL SOCIETY, WASHINGTON, DC, US, vol. 121, no. 6, 17 February 1999 (1999-02-17), pages 1399-1400, XP002146837 ISSN: 0002-7863
• BRINGLEY J F ET AL: "CONTROLLED CHEMICAL AND DRUG DELIVERY VIA THE INTERNAL AND EXTERNAL SURFACES OF LAYERED COMPOUNDS" JOURNAL OF DISPERSION SCIENCE AND TECHNOLOGY, NEW YORK, NY, US, vol. 24, no. 3/4, 2003, pages 589-605, XP009053587 ISSN: 0193-2691
• CHOY J-H ET AL: "INORGANIC LAYERED DOUBLE HYDROXIDES AS NONVIRAL VECTORS" ANGEWANDTE CHEMIE. INTERNATIONAL EDITION, WILEY VCH VERLAG, WEINHEIM, DE, vol. 39, no. 22, 17 November 2000 (2000-11-17), pages 4042-4045, XP000975901 ISSN: 1433-7851

## Description

The present invention relates to layered host materials with intercalated functional-active organic compounds. These materials are also for use in the delivery of functional-active organic compound to a target system.

## BACKGROUND OF THE INVENTION

The sequestration and controlled release of components in a chemical system can dramatically impact the performance and efficacy of a chemical system. For example, the controlled release of molecular components in photographic systems has long been used to improve the efficiency of photographic films. More recently, the sequestration and controlled release of health and cosmetically important molecules, such as fragrances, has been employed to improve the performance of consumer products. Sequestration refers to the ability of a complex system to first sequester or "hide" a specific component of the system, from a desired target component, system, or user. In the sequestrated form, the component may not interact, or react with the target system or component. The sequestered component may then later be released, either slowly through simple diffusion or by means of a chemical or physical switch applied to the system, to perform a desired function.

The most well known examples of sequestration and controlled release of components are in controlled drug-delivery. Controlled drug-delivery is important for treating disease and sickness in human and other plant and animal species. Much effort has been applied to the design and development of systems able to deliver a specific dose of a drug slowly over time to improve the efficacy of drugs and to decrease the risk of side effects to the patient. Primarily, the effort to date has been focused on encapsulating drugs within polymers and allowing for the drug to slowly diffuse through the polymeric layer into the patient. This has the problem in that the methods of producing such materials are expensive and often the release of the drug cannot be controlled, but rather relies on simple permeation and diffusion processes.

More recently, Choy et al. in U.S. patent 6,329,525 B1 have described a bio-inorganic composite for retaining and carrying bio-materials with stability and reversible dissociativity, and methods for preparing the bio-inorganic composite. The bio-inorganic hybrids are based upon intercalation of bio-molecules into layered host materials known as layered double hydroxides (often called anionic clays). The bio-molecules are adsorbed by the layered host material and ensconced therein, but later may be released upon exposure to a change in acidity or a change in electrolyte concentration. The bio-inorganic hybrids thus serve as a potentially effective means to effect controlled delivery of bio-materials such as drugs, health-care reagents and biomaterials.

WO patent application 02/47729 A2 to O'Hare describes a drug delivery system comprising an intercalate of a layered double hydroxide having, before intercalation, layers of metal hydroxides, and having intercalated therein a pharmaceutically-active compound having at least one anionic group. The intercalated compositions may be employed to release a variety of drugs within the stomach of a patient ingesting the "drug delivery system". The patent application also describes a method of preparing a layered double hydroxide having an intercalated pharmaceutically-active compound, which comprises treating an aqueous solution of the pharmaceutically-active compound with a layered double hydroxide where the pharmaceutically-active compound is present in a molar excess with respect to the layered double hydroxide, and separating the intercalate of the layered double hydroxide. The separation procedure generally involves filtration and washing of the product intercalate.

U.S. 2003/0129243 A1 to Pitard describes compositions comprising at least a nucleic acid and a mineral particle having an interchangeable foliate structure. The mineral particles employed are anionic clays (e.g., hydrotalcite) and the patent demonstrates aqueous compositions wherein, 80 - 90 % of the active chemistry (DNA in the example of Figure 5) is sequestered or removed from solution by the hydrotalcite, if the ratio of mineral (hydrotalcite) to DNA is greater than 50:1 (w/w).

There is a problem in the prior art in that the described compositions are expensive to prepare, and typically require a very large amount of layered host material to effectively sequester all the active material from solution. There is a further problem in that the compositions may release the active ingredient immediately upon exposure to an aqueous environment, including chemical or biological systems. There are still further problems in that the active components may be released prematurely or too quickly in the stomach of a patient and that the rate of release may be too high. There is a further problem in that the compositions may not readily form stable colloidal aqueous dispersions, as a result of the fact that there particle size may be too large, and accordingly may not be suitable for intravenous injection of a drug-delivery system.

## SUMMARY OF THE INVENTION

In accordance with one embodiment, the invention is directed towards an aqueous dispersion comprising particles of a layered host material and functional-active organic compound dispersed in an aqueous medium, wherein the weight ratio of layered host material to functional-active compound is less than 20 and at least 50% of the functional-active organic compound in the dispersion is intercalated between layers of the layered host material particles, and further wherein the aqueous medium comprises additional soluble salts or ionized molecules or polymers distinct from the functional-active compound and has an ionic strength of greater than 5 mS/cm. The invention is further directed towards an aqueous dispersion for use in the delivery of a desired functional-active organic compound to a target system, wherein the aqueous dispersion comprises the desired functional-active organic compound intercalated in a layered host material, and additional soluble salts or ionized molecules or polymers which are distinct from the functional-active compound and which are also present in the target system to which the functional-active compound is delivered.

## DETAILED DESCRIPTION OF THE INVENTION

In various embodiments, the invention relates to a composition for a chemical-delivery, and/or, a drug-delivery system, comprising an aqueous dispersion of a layered host material and an intercalated functional-active organic compound, wherein the weight ratio of layered host material to functional-active compound is less than 20 (preferably less than 10), and at least 50% (preferably at least 60%, more preferably at least 70%) of the functional-active compound in the composition resides between the layers of the layered host material. The aqueous medium also comprises additional soluble salts or ionized molecules or polymers distinct from the functional-active compound, and has an ionic strength of greater than 5 mS/cm (more preferably greater than 10 mS/cm). The term "functional-active" is used herein to refer to, e.g., chemically-active, pharmaceutically-active, or nutraceutically-active ions, molecules, complexes or polymers. In various further embodiments, the invention relates to an aqueous dispersion for use in the delivery of a desired functional-active organic compound to a target system, wherein the aqueous dispersion comprises the desired functional-active organic compound intercalated in a layered host material, and additional soluble salts or ionized molecules or polymers which are distinct from the functional-active compound and which are also present in the target system to which the functional-active compound is delivered. As the majority of the functional-active organic compound is intercalated in compositions in accordance with the invention, and as the compositions have a relatively high ionic strength, the invention provides a chemical-delivery and/or a drug-delivery system with significantly improved control over the rate of release of the functional-active component, especially for delivery of functional-active compounds in the stomach of a patient, and in a particular embodiment, control over the intravenous release of the intercalated functional-active component.

The chemical sequester and release (delivery) concepts of the invention are based upon intercalation chemistry. Intercalation is a process in which a layered material, referred to as the host, swells or opens to accommodate other molecules or ions, referred to as the guest.

$$\text{Host} \quad + \quad \text{guest} \quad \rightleftarrows \quad \text{Host(guest)}_x$$

Intercalation is, by definition, a reversible process and the guest molecules may diffuse from, or de-intercalate, from the interlayer space and are unaltered from the intercalation process. Layered compounds capable of sequestering ions and molecules by intercalation have been described in a number of publications. The choice of host material is dependent upon the particular molecule to be intercalated. A layered host material may be chosen which intercalates only cations, or conversely, only anions, or neutral molecules. The following publications are included for reference on this matter: "Intercalation Chemistry", A.J. Jacobson and S. Whittingham, eds., Academic Press, NY 1982; "Intercalated Layered Materials", F. Levy, D. Riedel Press, Dordrecht, Holland (1979); F. Trifiro and A. Vaccari, "Hydrotalcite-like anionic clays (Layered double hydroxides), in Comprehensive Supramolecular Chemistry, "Solid State Supramolecular Chemistry: Two- and Three-dimensional Inorganic Networks", Alberti G.; Bein, T. Eds., Elsevier, New York, Chapter 8 (1996); "An Introduction to Clay Colloid Chemistry", H. van Olphen, 2nd Ed., Krieger Pub. Co., Malabar, Fla. (1991).

Layered host materials for use in the present invention include:

1) Layered metal hydrogen phosphate structures of the formula:

$$M(HPO_4)_2 : yH_2O;$$

where M is Zr, Ti, Sn, Ge or Hf or any combination thereof; and y is a rational number between 0 and 10.

2) Layered double hydroxides of the general formulas:

$$[M^{2+}_{1-x} M^{3+}_{x}(OH)_2]^{x+} (x/n) A^{n-} \cdot yH_2O$$

or

$$[M^{1+}M^{3+}{}_2(OH)_6]^{1+}(1/n)A^{n-}\cdot yH_2O;$$

or hydroxy double salts of the general formula:

$$(M1^{2+}, M2^{2+})_5(OH)_8\cdot (A^{n-})_{2/n}\cdot yH_2O;$$

where $M^{1+}$ is a mono-valent metal which may be selected from, e.g., but not limited to Li, Na, K, Rb, Cs; $M^{2+}$, $M1^{2+}$, or $M2^{2+}$ is a divalent metal which may be selected from, e.g., but not limited to Ca, Mg, Mn, Co, Ni, Cu, Zn, Cd; and $M^{3+}$ is a trivalent metal which may be selected from, e.g., but not limited to Cr, Fe, Al, Ga, In, Mo; A is an inorganic or organic anion, including but limited to $OH^-$, $NO_3^-$, F, $Cl^-$, $Br^-$, $I^-$, $ClO_4^-$, $SO_4^{2-}$, $CO_3^{2-}$, chosen such that the rule of charge neutrality is obeyed; n is an integer; x may be any rational number between 0 and 1 (typically between 0.01 an 0.5, more typically between 0.1 and 0.4); and y may be any rational number between 0 and 10. Specific examples of layered double hydroxides include $Mg_2Al(OH)_6\cdot 1/2CO_3\cdot yH_2O$ (note that this is equivalent to $[Mg_{1-x}Al_x(OH)_2]$ $0.165\ CO_3\cdot yH_2O$ for x = 0.33); $Zn_2Al(OH)_6\cdot 1/2CO_3\cdot yH_2O$; $Mg_2Al(OH)_6\cdot NO_3\cdot yH_2O$; $Mg_2Al(OH)_6\cdot Cl\cdot yH_2O$; $Zn_2Al(OH)_6\cdot NO_3\cdot yH_2O$; $LiAl_2(OH)_6\cdot Cl\cdot yH_2O$ and $NaAl_2(OH)_6\cdot Cl\cdot yH_2O$.
3) Layered siliceous materials such as natural or synthetic clay minerals exemplified by montmorillonite, bentonite, kaolin, magadiite, hectorite, vermiculite, smectites, beidellite, fluorohectorite, talc, muscovite and saponite or given by the general formula:

$$[M1,M2]_nZ_4O_{10}(OH)_{2}\cdot_yH_2O\cdot_wM3;$$

where M1 is a metal selected from Al, Fe, Mn or Co and M2 is a metal selected from Mg, Fe, Ni, Zn or Li; Z is Al or Si; $H_2O$ is chemically absorbed water and M3 is a cation which may be selected from, but not limited to K, Na, Li or Ca. n is a number from 0 to 4, y is a number from 0 to 10 and w is a number from 0 to 1.

Intercalation of layered materials creates complex materials consisting of guest molecules or ions captured within the host matrix. The layers of the host solid, typically only a few angstroms thick, exfoliate and swell in direct proportion to the size of the guest molecules. The number of guest molecules captured within the layers is determined by their size and the charge of the guest and the host. The process is reversible such that the guest molecules or ions can later be recovered from the complex system.

As indicated above, the preferred choice of host material is dependent upon the particular molecule to be intercalated. In order to facilitate intercalation of a desired functional-active compound into a layered host material it may be necessary to prepare derivatives of the desired compound such that the compound attains a positive or negative charge. To prepare a cation, this may be typically achieved by derivatizing the functional-active compound with an onium ion group, e.g., an amine or quaternary amine, or phosphonium ion. To prepare an anion, a carboxylic or sulfonic acid function, or a sulfate group, may be attached to the parent functional-active compound.

The aqueous dispersion of the invention comprises an intercalated functional-active compound, and in a particular embodiment comprises a pharmaceutically-active compound. The pharmaceutically-active compound may include materials such as drugs, antibiotics, antimicrobials, bio-materials such as amino-acids, peptides, proteins, therapeutics, hormones, enzymes, growth factors, and genetic materials such as RNA, DNA, and oligonucleotides.

Because intercalation is a reversible process, release of an intercalated functional-active may occur immediately upon exposure of the intercalated complex to an environment having a high-ionic strength or a pH significantly different from that of the aqueous dispersion. The reversibility of the reaction has been exploited for controlled release of active chemistries and is discussed at length in U.S. patent 6,329,525 B1, W.O. patent application 02/47729 A2 and in docket 88101 co-filed herewith. The release process occurs through two general mechanisms, namely (i) ion-exchange and (ii) by a pH switch. The first release mechanism occurs through ion-exchange of the pharmaceutically-active (hereafter referred to as (P-A)) as shown in the following equation (for a layered host that intercalates anions):

$$\text{Host(P-A)}_x \ + \ M^+{}_{(aq.)}X^-{}_{(aq.)} \ \rightleftharpoons \ \text{Host(X}^-)_x \ + \ (\text{P-A})^-{}_{(aq.)}M^+{}_{(aq.)}$$

The process is virtually identical for cationic (P-A), with the exception that the cation ($M^+$) is exchanged for the P-A. The process is an equilibrium process and addition of salts or ions may drive the equilibrium of the above equation to the right by Le Chatelier's principle. The release of the P-A is therefore driven by a gradient in ionic-strength between the intercalated composition and the surrounding environment. Therefore, when an intercalated layered material, or dispersion comprising an intercalated layered material, is placed in contact with a target environment (such as a physiological environment, e.g., blood, stomach, mucous, etc.) release via ion-exchange will commence immediately providing that the ionic-strength of the target environment is greater than the ionic-strength of the initial environment.

In many cases, it is desirable to control release of the P-A, e.g. to time delay release or to target release into only a very specific target, such as cells or tumors. To facilitate control of the rate of release of the functional-active from the interlayer space of the layered host materials, the aqueous dispersion of the invention contains, in addition to the layered host material and the desired functional-active compound, additional soluble salts or ionized molecules or polymers distinct from the functional-active compound, and has an ionic strength of greater than 5 mS/cm (more preferably greater than 10 mS/cm. It is preferred that some of the additional ions are co-intercalated into the layered host material. Additional intercalated ions may form intercalated compositions having the general formula:

$$\text{Host(P-A)}_x \, \text{(A-I)}_y \, ;$$

where A-1 represent the additional ions. It is preferred that the additional ions are chosen such that they comprise the ions present in the target system in which the inventive functional-active compound delivery composition is used. For example, human blood is highly saline, containing 0.9 weight percent NaCl. In this case, it is preferred that the additional ions comprise chloride ions (for a layered host that intercalates anionic P-A's) and Na-ions (for a layered host that intercalates cationic P-A's).

The additional ions of the inventive composition serve to reduce the ionic-strength gradient that the intercalated composition may experience when placed into the target system, and the rate of release (equilibrium in above equation) is controlled. It is preferred that the inventive aqueous dispersion has an ionic-strength greater than 10 mS/cm. It is further preferred that the inventive aqueous dispersion has an ionic-strength equal to or greater than the ionic-strength of the target system in which the inventive composition is employed. In this case, there is no driving force (ionic-strength gradient) for release of the P-A via ion-exchange, and thus the secondary mechanism of pH change may be used to controllably release the P-A to a significant extent. As pointed out by Choy et al. (U.S. Patent 6,329,525 B1), a pH change release mechanism may be used to release the P-A directly into cells (such as tumor cells) since the pH inside of cells is considerably more acidic than physiological pH (pH 7.4). By matching the ionic-strength of physiological conditions, the intercalated compositions of the invention essentially turns off, or significantly diminishes, release of P-A due to ion-exchange, and allows only the pH release mechanism to be operative. This method of limiting the release of P-A to only one mechanism thus facilitates the targeted delivery of the P-A directly to cells and cell tissue, and to tumor cells.

In addition to being selected based on the ionic composition of a target system, the additional ions employed int eh aqueous dispersion must be selected based on the type of layered host material employed. For layered host materials comprising a layered double hydroxide or a hydroxy double salt, the additional soluble salts or ionized molecules or polymers distinct from the functional-active compound ions may preferably comprise Cl⁻, $NO_3^-$, phosphates, sulfates or negatively charged amino acids such as aspartic acid or glutamic acid. For layered host material comprising a metal hydrogen phosphate, clay or layered silicate, the additional soluble salts or ionized molecules or polymers distinct from the functional-active compound ions may preferably comprise $Na^+$, $K^+$, $Ca^{++}$, $Mg^{++}$, or positively charged amino acids such as lysine, arginine, or histidine.

The aqueous dispersions of the invention comprise layered host material and functional-active organic compound wherein the weight ratio of layered host material to functional-active compound is less than 20 (preferably less than 10), and at least 50% (preferably at least 60%, more preferably at least 70%) of the functional-active compound in the composition resides between the layers of the layered host material. Such combination of sufficiently high intercalation (high loading) percentage and relatively low ratio of layered host requirements assure that the dispersions enable efficient and controllable delivery of the functional-active compound without a need for high levels of layered host material. In accordance with one particular embodiment, layered host materials in nanoparticulate form (e.g., having a mean, volume weighted, particle size of less than 500 nm, preferably less than 200 nm, and most preferably less than 100 nm) may be used to facilitate high intercalation percentages at relatively low layered host material levels. Cationic clays are commercially available as nanoparticulates under the tradename Laponite, e.g., and such nanoparticulate cationic clays may be used as layered host materials in combination with cationic functional-active compounds. In accordance with a further specific embodiment of the invention, nanoparticulate anionic clays prepared in accordance with commonly assigned, concurrently filed, copending USSN 10/850,489, may be used in combination with anionic functional-active compounds. As disclosed therein, nanoparticulate anionic clay particle compositions and colloidal dispersions for use in accordance with such embodiment of the invention may be prepared by a process comprising simultaneously bringing together salt solutions of $M^{2+}$ and $M^{3+}$ metals, or of $M^{1+}$ and $M^{3+}$ metals, and a base in a high shear mixing zone within a dispersion medium in a particle precipitation vessel.

The aqueous dispersion of the invention in accordance with one embodiment provides an intercalated functional-active molecule or polymer, and additionally salts or ionized molecules or polymers, wherein the dispersion is a stable colloidal dispersion having an average mean, volume weighted, particle diameter of less than 500 nm. A stable colloid as referenced in the examples is defined as a particulate suspension in which there is no evidence for aggregation of particles as determined by particle size measurement, and that there is not visible flocculation or settling of the colloid for an extended period (e.g., at least one week) after its preparation. Particle growth due to aggregation of the primary particles to average particle size diameters greater than four times the original diameter, and visible settling of the colloid within one week of its preparation is indicative of an unstable colloid. The stable colloid further preferably does not aggregate under physiological conditions and thus may be homogeneously dispersed in the bloodstream, or in mucous membranes. The stable colloid has an average particle diameter of less than 500 nm. Particulates of this size are suitable for intravenous injection, or application via mucous membranes. Particulates significantly larger than 500 nm are not suitable for such applications. It is preferred that the intercalated particles of the invention have an average particle diameter of less than 200 nm, and more preferably less than 100 nm. Particles of such small diameters may pass through the walls of blood vessels and especially through the walls of blood vessels in close proximity to tumors, by the EPR effect. The advantages of nanoparticulate carriers have been discussed by Moghimi et al. Pharmacological Reviews, 53, 283 (2001).

High percentages of intercalation for functional-active compounds at relatively low layered host material levels may also be facilitated though preparation of the intercalated compound dispersion via "reconstruction synthesis" of layered double hydroxide material from a calcined product of an anionic clay, such as taught in commonly assigned, copending USSN 10/837,189, filed April 30, 2004. The term "calcination" refers to a heating process whereby a layered double hydroxide, or hydrotalcite, is heated to decomposition usually at a temperature greater than 300 °C. The thermal treatment of hydrotalcite produces a calcined product according to the following reaction:

$$Mg_{0.7}Al_{0.3}(OH)_2 \cdot 0.15CO_3 \cdot yH_2O \text{ --------> } 1.15 \, Mg_{0.62}Al_{0.26}O$$

The calcined product is an amorphous mixed oxide and when placed in water, even at room temperature, has a strong tendency to redydrate and will reform a layered double hydroxide, if a suitable anion is present. This unusual property of the calcined layered double hydroxide is often referred to as "reconstruction synthesis". The reconstruction of a layered double hydroxide with a nitrate anion is depicted in the following equation:

$$1.15 \, Mg_{0.62}Al_{0.26}O + 0.3 \, HNO_3 \text{ -----> } Mg_{0.7}Al_{0.3}(OH)_2 \cdot 0.3 \, NO_3 \cdot yH_2O$$

In general, the reconstruction of a layered double hydroxide may be accomplished according to the following equation:

$$1.15 \, Mg_{0.62}Al_{0.26}O + 0.3/n \, H^+ \, Anion^{-n} \text{ -----> } Mg_{0.7}Al_{0.3}(OH)_2 \cdot 0.3/n \, Anion \cdot yH_2O$$

The amorphous solid is re-dispersed in aqueous media containing a suitable organic or inorganic anion, and may "reconstruct" to form the layered intercalation compound of the new anion. The "new" anionic species may be chosen such that it imparts a particular chemical, pharmaceutical or biological function, and represents the "functional-active" of the invention. The reaction has the stoichiometry as indicated in Eq. (6) and generally proceeds readily at, or just above, room temperature. The reaction is quite remarkable in that in many cases it proceeds to completion and has no by-products. Calcined derivatives of layered double hydroxides are therefore preferred because they provide aqueous chemical-delivery, and/or, a drug-delivery systems which do not require filtration and washing, and are simple and inexpensive to prepare.

In such reconstruction process, the calcined layered double hydroxide, hereafter referred to as c-LDH, is provided in aqueous dispersion in a stoichiometric ratio in excess of that necessary (as calculated from the molar ion-exchange capacity) to react and intercalate the functional-active. The reconstruction intercalation thus requires the addition of a secondary anion, such as nitrate, chloride, bromide or perchlorate ($ClO_4^-$), for charge neutrality. The additional soluble salts or ionized molecules or polymers distinct from the functional-active compound provided in the compositions of the invention may be conveniently used as the secondary anion, and be co-intercalated with the functional-active compound during reconstruction of calcined layered double hydroxide. The reaction may be described as below for anionic clays of the first type:

$$[M^{2+}_{1-x} M^{3+}_{x} O_{1+x/2}] \ + \ a/n \ (F\text{-}A)^{n-} \ + \ b/p \ (anion2)^{p-}$$

$$\updownarrow$$

$$[M^{2+}_{1-x} M^{3+}_{x} (OH)_2] \ a/n \ (F\text{-}A)^{n-} \ b/p \ (anion2)^{p-} \cdot yH_2O;$$

where $(n)(a) + (p)(b) = x$, and $(p)(b)/(n)(a)$ is preferably between 0.75 and 5.0. It is preferred that the anion2 is provided in the form of an acid such as $HNO_3$, HCl, HBr or $HClO_4$. It is further preferred that the pH of the reaction mixture is controlled between 5 and 9. This is preferred because the intercalation reaction is pH dependent, and de-intercalation may occur at pH values outside of this range (absent countervailing measures).

Compositions in accordance with various embodiments of the invention optionally may further comprise a polymer. The polymer may be employed, e.g., to further slow the rate of release of the functional active from the layered host material particles. The polymer may be used to encapsulate the composition of matter into a pill or other ingestible tablet or capsule. It is preferred that the polymer is a biocompatible polymer. Polymer suitable for practice of the invention include polyethyleneglycol, methoxypolyethylene glycol, polypropylene glycol, dextran, polylysine, polysaccharides, polypeptides, gelatin, albumin, chitosan, cellulose, hydrogels, polyvinyl alcohol, water-based polyurethanes, polyester, nylon, high nitrile resins, polyethylene-polyvinyl alcohol copolymer, polystyrene, ethyl cellulose, cellulose acetate, cellulose nitrate, aqueous latexes, polyacrylic acid, polystyrene sulfonate, polyamide, polymethacrylate, polyethylene terephthalate, polystyrene, polyethylene, polypropylene or polyacrylonitrile.

**EXAMPLES**

**Examples 1.1-1.6: LDH and salicyclic acid**

The following examples compare the sequestration efficiency of intercalated dispersions prepared at high ionic strength (inventive examples) versus those prepared at low ionic strength (comparison examples). Hydrotalcite (Layered double hydroxide) was obtained from Sud Chemie having the composition $Mg_{0.7}Al_{0.3}(OH)_2 \cdot 0.15 \ CO_3 \cdot 0.6H_2O$. This material was calcined before use at 500°C for 3 h and cooled in a nitrogen atmosphere, the calcined product is hereafter referred to as c-LDH and has the approximate composition $Mg_{0.62}Al_{0.26}O$. Distilled water employed in the Examples was boiled prior to use to eliminate dissolved carbon dioxide.

**Comparison Example 1.1:** 0.50 g of salicylic acid and 4.0 equivalents (2.16 g) of c-LDH were combined in 50.00 ml of distilled water, and the pH adjusted to between 7.0 and 7.5. The ionic strength of the reaction mixture was 1.4 mS/cm. The suspension was tightly sealed from the atmosphere and allowed to stir overnight at 50 °C. 1.00 g of the suspension was then added to 9.00 ml of a 0.9 % aqueous NaCl solution. To determine the sequestration efficiency, the resulting diluted dispersion was centrifuged at 7500 rpm and an aliquot of the supernatant was examined by liquid chromatography, LC. The analysis give the concentration of un-intercalated guest in the supernatant. The percent uptake of the guest (functional-active) by the host was calculated by as follows:

$$\% \ uptake = (nominal \ guest \ conc. \ - \ found \ conc.)/(nominal \ guest \ conc.)$$

The results are reported in Table 1.

**Comparison Example 1.2:** 0.50 g of salicylic acid and 6.0 equivalents (3.24 g) of c-LDH were combined in 50.00 ml of distilled water, and the pH adjusted to 7.0. The ionic strength of the reaction mixture was 2.1 mS/cm. The suspension was tightly sealed from the atmosphere and allowed to stir overnight at 50°C. 1.00 g of the suspension was then added to 9.00 ml of a 0.9 % aqueous NaCl solution and analyzed as defined above; the results are given in Table 1.

**Comparison Example 1.3:** 0.50 g of salicylic acid and 8.0 equivalents (4.31 g) of c-LDH were combined in 50.00 ml of distilled water, and the pH adjusted to 7.0. The ionic strength of the reaction mixture was 2.4 mS/cm. The suspension was tightly sealed from the atmosphere and allowed to stir overnight at 50 °C. 1.00 g of the suspension was then added to 9.00 ml of a 0.9 % aqueous NaCl solution and analyzed as defined above; the results are given in Table 1.

**Example 1.4:** 0.50 g of salicylic acid and 4.0 equivalents (2.16 g) of c-LDH were combined in 50.00 g of an aqueous 0.9 % NaCl solution, and the pH adjusted to between 7.0 and 7.5. The ionic strength of the reaction mixture was 14.3 mS/cm. The suspension was tightly sealed from the atmosphere and allowed to stir overnight at 50 °C. The suspension was tightly sealed from the atmosphere and allowed to stir overnight at 50 °C. 1.00 g of the suspension was then added to 9.00 ml of a 0.9 % aqueous NaCl solution and analyzed as defined above; the results are given in Table 1.

**Example 1.5:** 0.50 g of salicylic acid and 6.0 equivalents (3.24 g) of c-LDH were combined in 50.00 g of an aqueous 0.9 % NaCl solution, and the pH adjusted to between 7.0 and 7.5. The ionic strength of the reaction mixture was 14.6 mS/cm. The suspension was tightly sealed from the atmosphere and allowed to stir overnight at 50°C. 1.00 g of the suspension was then added to 9.00 ml of a 0.9 % aqueous NaCl solution and analyzed as defined above; the results are given in Table 1.

**Example 1.6:** 0.50 g of salicylic acid and 8.0 equivalents (4.31 g) of c-LDH were combined in 50.00 g of an aqueous 0.9 % NaCl solution, and the pH adjusted to between 7.0 and 7.5. The ionic strength of the reaction mixture was 15.6 mS/cm. The suspension was tightly sealed from the atmosphere and allowed to stir overnight at 50 °C. 1.00 g of the suspension was then added to 9.00 ml of a 0.9 % aqueous NaCl solution and analyzed as defined above; the results are given in Table 1.

Table 1. Comparison of the sequestration efficiency of intercalated dispersions prepared at high ionic strength (inventive examples) versus those prepared at low ionic strength (comparative examples).

| Example (E) or Comparison example (CE) | Ionic strength at preparation | stoich. ratio c-LDH:F-A | sequestration efficiency upon dilution |
|---|---|---|---|
| CE-1.1 | 1.4 | 4.0 | 6 |
| CE-1.2 | 2.1 | 6.0 | 15 |
| CE-1.3 | 2.4 | 8.0 | 21 |
| E-1.4 | 14.3 | 4.0 | 51 |
| E-1.5 | 14.6 | 6.0 | 54 |
| E-1.6 | 15.6 | 8.0 | 64 |

The data of Table 1 indicate that the compositions prepared by the inventive method have a significantly improved sequestration efficiency of the functional-active compound. The degree of sequestration is poor for all comparison examples even when the stoichiometric ratio of the hydrotalcite is very high.

**Examples 2.1-2.3: Nanoparticle LDH colloids and Oligonucleotides**

The following examples demonstrate efficient sequestration of oligonucleotides at high-ionic strength by a pristine nanoparticulate LDH colloid at desirably low weight ratio of LDH to oligonucleotides.

Preparation of LDH anionic clay colloid: Into a 3.0 L vessel containing 400 ml of distilled water which was stirred with a highly efficient prop-like mixing apparatus of the type described in Research Disclosure, Vol. 382, February 1996, Item 38213, employed at a rate of 2000 rpm was simultaneously added, 650.0 ml of a 2.0 M $MgCl_2:6H_2O$ solution at 50 ml per minute for 13 minutes; 650.0 ml of a 1.0 M $AlCl_3:6H_2O$ solution at 50 ml per minute for 13 minutes; and a 5.0 M NaOH solution at a rate sufficient to keep the pH of the reaction mixture pinned between 8.5 - 9.0. The addition rate of 5.0 NaOH varied slightly between about 50 and 55 ml/min. After the addition was completed, the dispersion was then washed free of salt by dialfiltration, until the ionic conductivity of the dispersion was less than about 0.1 mS. The resulting dispersion had a volume weighted mean particle size diameter of 23 nm with a standard deviation of 10 nm, and did not settle after standing one week indicating that the dispersion was a stable colloid. The 50 percentile average particle size was 23 nm (i.e., 50 % of the particles are less than 23 nm in size) and 95 % of the particles were less than 55 nm. The percent solids of the final dispersion was 2.4 % by weight.

For Examples 2.1-2.3, an oligonucleotide (Hp C probe) was obtained from Integrated DNA Technologies, Inc. with a molecular weight of 6,361 Da. A solution of the oligonucleotide was prepared having a concentration of 40.0 μg/ml oligonucleotide and 0.9 weight percent NaCl (ionic strength = 13.7 mS/cm); the optical density of this solution at 260 nm was 1.2.

Distilled water employed in the Examples was boiled prior to use to eliminate dissolved carbon dioxide.

**Example 2.1.** Into 3.0 ml of the oligonucleotide solution (40.0 μg/ml) was added 5.0 μL (0.005 ml) of the clay colloid prepared as above. The suspension was allowed to stand for 18 h and then the suspension was diafiltered through a 30,000 molecular weight cutoff filter using a beckman microcentrifuge apparatus. The filtrate was then examined by visible spectroscopy and the percent of intercalated oligonucleotide was determined as follows: 100 % x [1.20 - measured O.D]/1.20. The results are reported in Table 2.

**Example 2.2.** Into 3.0 ml of the oligonucleotide solution (40.0 μg/ml) was added 10.0 μL (0.005 ml) of the clay colloid prepared as above. The suspension was allowed to stand for 18 h and then the suspension was diafiltered through a 30,000 molecular weight cutoff filter using a beckman microcentrifuge apparatus. The filtrate was then examined by visible spectroscopy and the percent of intercalated oligonucleotide was determined as follows: 100 % x [1.20 - measured O.D]/1.20. The results are reported in Table 2.

**Example 2.3.** Into 3.0 ml of the oligonucleotide solution (40.0 μg/ml) was added 25.0 μL (0.005 ml) of the clay colloid prepared as above. The suspension was allowed to stand for 18 h and then the suspension was diafiltered through a 30,000 molecular weight cutoff filter using a beckman microcentrifuge apparatus. The filtrate was then examined by visible spectroscopy and the percent of intercalated oligonucleotide was determined as follows: 100 % x [1.20 - measured O.D]/1.20. The results are reported in Table 2.

**Table 2.**

| Example | weight ratio of clay colloid:ologinucleotide | % uptake oligonucleotide |
|---------|---------------------------------------------|--------------------------|
| E-2.1   | 1.0                                         | 55                       |
| E-2:2   | 2.0                                         | 69                       |
| E-2.3   | 5.0                                         | 80                       |

The data of Table 2 demonstrate that the sequestration efficiency of an oligonucleotide is advantageously very high for the nanoparticulate colloid added to the high ionic strength oligonucleotide solution, even at relatively low weight ratios of colloid to oligonucleotide.

**Claims**

1.  An aqueous dispersion comprising particles of a layered host material and functional-active organic compound dispersed in an aqueous medium, wherein the weight ratio of layered host material to functional-active compound is less than 20 and at least 50% of the functional-active organic compound in the dispersion is intercalated between layers of the layered host material particles, and further wherein the aqueous medium comprises additional soluble salts or ionized molecules or polymers distinct from the functional-active compound and has an ionic strength of greater than 5 mS/cm.

2.  An aqueous dispersion according to claim 1, wherein the functional-active compound comprises a pharmaceutically-active molecule or polymer.

3.  An aqueous dispersion according to claim 1 wherein the functional-active comprises a drug, antibiotic, antimicrobial, amino-acid, peptide, protein, therapeutic, hormone, enzyme, growth factor, RNA, DNA, or oligonucleotide compound.

4.  An aqueous dispersion according to claim 1, wherein the dispersion comprises stable colloidal dispersed layered host material particles having an average mean, volume weighted, particle diameter of less than 500 nm.

5.  An aqueous dispersion according to claim 4, wherein the layered host material prior to intercalation of the functional-active compound comprises a layered double hydroxide of the general formulas:

$$[M^{2+}_{1-x} M^{3+}_x (OH)_2]^{x+} (x/n) A^{n-} \cdot yH_2O$$

or

$$[M^{1+} M^{3+}_2 (OH)_6]^{1+} (1/n) A^{n-} \cdot yH_2O;$$

where $M^{1+}$ is a mono-valent metal; $M^{2+}$ is a divalent metal; and $M^{3+}$ is a trivalent metal; A is an inorganic or organic anion, chosen such that the rule of charge neutrality is obeyed; n is an integer; x is any rational number between 0 and 1; and y is any rational number between 0 and 10.

6.  An aqueous dispersion according to claim 1, wherein the layered host material is reconstructed from a calcined product of $Mg_2Al(OH)_6 \cdot 1/2CO_3 \cdot yH_2O$, $Zn_2Al(OH)_6 \cdot 1/2CO_3 \cdot yH_2O$, $Mg_2Al(OH)_6 \cdot NO_3 \cdot yH_2O$ or $Zn_2Al(OH)_6 \cdot NO_3 \cdot yH_2O$.

7. An aqueous dispersion according to claim 1, wherein the layered host material comprises a layered double hydroxide or a hydroxy double salt, and the additional soluble salts or ionized molecules or polymers distinct from the functional-active compound ions comprise $Cl^-$, $NO_3^-$, phosphates, sulfates or negatively charged amino acids such as aspartic acid or glutamic acid.

8. An aqueous dispersion according to claim 1, wherein the layered host material comprises a metal hydrogen phosphate, clay or layered silicate, and the additional soluble salts or ionized molecules or polymers distinct from the functional-active compound ions comprise $Na^+$, $K^+$, $Ca^{++}$, $Mg^{++}$, or positively charged amino acids such as lysine, arginine, or histidine.

9. An aqueous dispersion according to claim 1 for use in the delivery of a desired functional-active organic compound to a target system, wherein the aqueous dispersion comprises the desired functional-active organic compound intercalated in a layered host material, and additional soluble salts or ionized molecules or polymers which are distinct from the functional-active compound and which are also present in the target system to which the functional-active compound is delivered.

10. An aqueous dispersion according to claim 9, wherein the target system is a biological system and the additional soluble salts or ionized molecules or polymers which are distinct from the functional-active compound comprises sodium or chloride ion.


**Patentansprüche**

1. Wässrige Dispersion mit Teilchen eines geschichteten Wirtsmaterials und einer in einem wässrigen Medium dispergierten funktionsaktiven, organischen Verbindung, worin das Gewichtsverhältnis des geschichteten Wirtsmaterials zur funktionsaktiven Verbindung kleiner als 20 ist, und worin mindestens 50% der funktionsaktiven, organischen Verbindung in der Dispersion zwischen Schichten der geschichteten Wirtsmaterialteilchen eingelagert sind, und worin das wässrige Medium zusätzliche lösliche Salze oder ionisierte Moleküle oder Polymere umfasst, die sich von der funktionsaktiven Verbindung unterscheiden, und eine Ionenstärke von größer als 5 mS/cm aufweist.

2. Wässrige Dispersion nach Anspruch 1, worin die funktionsaktive Verbindung ein pharmazeutisch aktives Molekül oder Polymer umfasst.

3. Wässrige Dispersion nach Anspruch 1, worin die funktionsaktive Verbindung eine pharmazeutische, antibiotische, antimikrobielle, Aminosäure-, Peptid-, Protein-, therapeutische, Hormon-, Enzym-, Wachstumsfaktor-, RNA-, DNA-, oder Oligonukleotid-Verbindung umfasst.

4. Wässrige Dispersion nach Anspruch 1, worin die Dispersion stabile, kolloidale, dispergierte, geschichtete Wirtsmaterialteilchen mit einem mittleren, volumengewichteten Teilchendurchmesser von kleiner als 500 nm umfasst.

5. Wässrige Dispersion nach Anspruch 4, worin das geschichtete Wirtsmaterial vor Einlagerung der funktionsaktiven Verbindung ein geschichtetes Doppelhydroxid der folgenden allgemeinen Formeln umfasst:

$$[M^{2+}_{1-x} M^{3+}_x (OH)_2]^{x+} (x/n) A^{n-} \cdot yH_2O$$

oder

$$[M^{1+} M^{3+}_2 (OH)_6]^{1+} (1/n) A^{n-} \cdot yH_2O;$$

wobei $M^{1+}$ für ein einwertiges Metall; $M^{2+}$ für ein zweiwertiges Metall; und $M^{3+}$ für ein dreiwertiges Metall steht; A für ein anorganisches oder organisches Anion steht, das unter Beachtung der Regel der Ladungsneutralität ausgewählt ist; n für eine ganze Zahl; x für eine beliebige rationale Zahl zwischen 0 und 1 und y für eine beliebige rationale Zahl zwischen 0 und 10 steht.

6. Wässrige Dispersion nach Anspruch 1, worin das geschichtete Wirtsmaterial aus einem kalzinierten Produkt aus $Mg_2Al(OH)_6 \cdot 1/2CO_3 \cdot yH_2O$, $Zn_2Al(OH)_6 \cdot 1/2CO_3 \cdot yH_2O$, $Mg_2Al(OH)_6 \cdot NO_3 \cdot yH_2O$ oder $Zn_2Al(OH)_6 \cdot NO_3 \cdot yH_2O$ wiederaufgebaut ist.

**7.** Wässrige Dispersion nach Anspruch 1, worin das geschichtete Wirtsmaterial ein geschichtetes Doppelhydroxid oder ein Hydroxydoppelsalz umfasst, und worin die zusätzlichen löslichen Salze oder ionisierten Moleküle oder Polymere, die sich von Ionen der funktionsaktiven Verbindung unterscheiden, Cl⁻, $NO_3^-$, Phosphate, Sulfate oder negativ geladene Aminosäuren wie Asparaginsäure oder Glutaminsäure umfassen.

**8.** Wässrige Dispersion nach Anspruch 1, worin das geschichtete Wirtsmaterial ein Metall-Wasserstoffphosphat, Ton oder geschichtetes Silicat umfasst, und die zusätzlichen löslichen Salze oder ionisierten Moleküle oder Polymere, die sich von den Ionen der funktionsaktiven Verbindung unterscheiden, $Na^+$, $K^+$, $Ca^{++}$, $Mg^{++}$ oder positiv geladene Aminosäuren wie Lysin, Arginin oder Histidin umfassen.

**9.** Wässrige Dispersion nach Anspruch 1 zur Verwendung in der Bereitstellung einer gewünschten funktionsaktiven, organischen Verbindung für ein Zielsystem, worin die wässrige Dispersion die gewünschte funktionsaktive, organische Verbindung umfasst, die in einem geschichteten Wirtsmaterial eingelagert ist, und zusätzliche, lösliche Salze oder ionisierte Moleküle oder Polymere, die sich von der funktionsaktiven Verbindung unterscheiden, und die ebenfalls in dem Zielsystem vorhanden sind, dem die funktionsaktive Verbindung bereitgestellt wird.

**10.** Wässrige Dispersion nach Anspruch 9, worin das Zielsystem ein biologisches System ist und die zusätzlichen löslichen Salze oder ionisierten Moleküle oder Polymere, die sich von der funktionsaktiven Verbindung unterscheiden, Natrium- oder Chloridionen umfassen.

**Revendications**

**1.** Dispersion aqueuse comprenant des particules d'un matériau hôte stratifié et un composé organique actif fonctionnel dispersé dans un milieu aqueux, dans laquelle le rapport en poids du matériau hôte stratifié au composé actif fonctionnel est inférieur à 20 et au moins 50% du composé organique actif fonctionnel de la dispersion est intercalé entre les couches des particules du matériau hôte stratifié, et dans laquelle en outre le milieu aqueux comprend d'autres sels solubles ou molécules ou polymères ionisés distincts du composé actif fonctionnel et a une force ionique supérieure à 5 mS/cm.

**2.** Dispersion aqueuse selon la revendication 1, dans laquelle le composé actif fonctionnel comprend une molécule ou un polymère pharmaceutiquement actif.

**3.** Dispersion aqueuse selon la revendication 1, dans laquelle le composé actif fonctionnel comprend un médicament, un antibiotique, un antimicrobien, un acide aminé, un peptide, une protéine, une substance thérapeutique, une hormone, un enzyme, un facteur de croissance, de l'ARN, de l'ADN ou un composé oligonucléotide.

**4.** Dispersion aqueuse selon la revendication 1, dans laquelle la dispersion comprend des particules du matériau hôte stratifié dispersées dans une substance colloïdale stable ayant un diamètre moyen de particule pondéré en volume inférieur à 500 nm.

**5.** Dispersion aqueuse selon la revendication 4, dans laquelle le matériau hôte stratifié, avant d'y intercaler le composé actif fonctionnel, comprend un hydroxyde double stratifié représenté par les formules générales suivantes :

$$[M^{2+}_{1-x} M^{3+}_x (OH)_2]^{x+} (x/n)A^{n-}.yH_2O$$

ou

$$[M^{1+} M^{3+}_2 (OH)_6]^{1+} (1/n)A^{n-}.yH_2O ;$$

où $M^{1+}$ est un métal monovalent ; $M^{2+}$ est un métal divalent et $M^{3+}$ est un métal trivalent ; A est un anion organique ou inorganique, sélectionné de sorte que la règle de la neutralité de charge soit respectée ; n est un entier ; x est un nombre rationnel quelconque compris entre 0 et 1 ; et y est un nombre rationnel quelconque compris entre 0 et 10.

**6.** Dispersion aqueuse selon la revendication 1, dans laquelle le matériau hôte stratifié est reconstruit à partir d'un produit calciné de $Mg_2Al(OH)_6 \cdot 1/2CO_3 \cdot yH_2O$, $Zn_2Al(OH)_6 \cdot 1/2CO_3 \cdot yH_2O$, $Mg_2Al(OH)_6 \cdot NO_3 \cdot yH_2O$ ou $Zn_2Al(OH)_6 \cdot NO_3 \cdot yH_2O$.

**7.** Dispersion aqueuse selon la revendication 1, dans laquelle le matériau hôte stratifié comprend un hydroxyde double stratifié ou un sel double d'hydroxy et les autres sels solubles ou molécules ou polymères ionisés distincts des ions du composé actif fonctionnel comprennent Cl$^-$, NO$_3^-$, les phosphates, les sulfates ou les acides aminés de charge négative, tels que l'acide aspartique ou l'acide glutamique.

**8.** Dispersion aqueuse selon la revendication 1, dans laquelle le matériau hôte stratifié comprend un phosphate hydrogéné métallique, de l'argile ou du silicate stratifié et les autres sels solubles ou molécules ou polymères ionisés distincts des ions du composé actif fonctionnel comprennent Na$^+$, K$^+$, Ca$^{++}$, Mg$^{++}$ ou des acides aminés de charge positive, tels que la lysine, l'arginine ou l'histidine.

**9.** Dispersion aqueuse selon la revendication 1 utilisée pour administrer un composé organique actif fonctionnel désiré à un système cible, dans laquelle la dispersion aqueuse comprend le composé organique actif fonctionnel désiré intercalé dans un matériau hôte stratifié, et d'autres sels solubles ou molécules ou polymères ionisés qui sont distincts du composé actif fonctionnel et qui sont également présents dans le système cible auquel le composé actif fonctionnel est administré.

**10.** Dispersion aqueuse selon la revendication 9, dans laquelle le système cible est un système biologique et les autres sels solubles ou molécules ou polymères ionisés distincts du composé actif fonctionnel comprennent l'ion sodium ou l'ion chlorure.

## REFERENCES CITED IN THE DESCRIPTION

### Patent documents cited in the description

- US 6329525 B1, Choy
- WO 0247729 A2, O'Hare
- US 20030129243 A1, Pitard
- US SN10850489 A
- US 10837189 B

### Non-patent literature cited in the description

- Intercalation Chemistry. Academic Press, 1982
- Intercalated Layered Materials. 1979
- Hydrotalcite-like anionic clays. **F. TRIFIRO ; A. VACCARI.** Comprehensive Supramolecular Chemistry
- Solid State Supramolecular Chemistry: Two- and Three-dimensional Inorganic Networks. Elsevier, 1996
- An Introduction to Clay Colloid Chemistry. Krieger Pub. Co, 1991
- **MOGHIMI et al.** *Pharmacological Reviews,* 2001, vol. 53, 283
- *Research Disclosure,* February 1996, vol. 382